# EUROPÄISCHE PATENTSCHRIFT

(11) **EP 2 011 523 B1**
(45) Veröffentlichungstag und Bekanntmachung des Hinweises auf die Patenterteilung: **27.11.2013**
(21) Anmeldenummer: 08008966.7
(22) Anmeldetag: 15.05.2008
(51) Int. Cl.: A61L 15/32, A61L 26/00, A61L 31/04

(54) **Blutstillendes Vlies**
Blood-stopping non-woven fabric
Etoffe hémostatique

(30) Priorität: 15.05.2007 DE 102007024220
(43) Veröffentlichungstag der Anmeldung: 07.01.2009
(73) Patentinhaber: Aesculap AG, 78532 Tuttlingen (DE)
(72) Erfinder: Wegmann, Jürgen, 78333 Stockach (DE); Odermatt, Erich, 8200 Schaffhausen (CH)
(74) Vertreter: Patentanwälte Ruff, Wilhelm, Beier, Dauster & Partner

(56) Entgegenhaltungen:
- WO-A-00/38752
- WO-A-2006/088912
- WO-A-2006/102457
- DE-U1-202005 020 566
- GB-A- 1 331 964
- US-A1- 2005 095 275
- US-A1- 2005 123 588
- US-A1- 2007 009 736

## Beschreibung

Die Erfindung betrifft ein Vlies, insbesondere zur Blutstillung, ein Verfahren zur Herstellung des Vlieses, verschiedene Verwendungen des Vlieses sowie ein hämostatisches Mittel.

Die rasche und insbesondere gewebschonende Blutstillung (Hämostase) von Wunden aller Art ist ein zentrales Anliegen der modernen Versorgungsmedizin. Die zum Einsatz kommenden Hämostatika müssen demnach eine ausreichende Aufnahmekapazität für Körperflüssigkeiten, insbesondere für Blut, aufweisen. Dadurch werden die Blutplättchen im Wundmilieu angereichert und die Blutgerinnung beschleunigt.

In der Zwischenzeit kommen unzählige blutstillende Mittel zum Einsatz. Geeignete Wundverbände sind beispielsweise aus der WO 03/086234 A2, WO 02/40242 A1, US 4,043,331 bekannt. In der WO 2006/088912 A2 sind blutstillende Mittel beschrieben, welche Tonmineralien enthalten. Nachteilig hierbei ist jedoch, dass Tonmineralien gewöhnlich nicht resorbierbar sind und sich daher weniger zur Blutstillung von inneren Wunden eignen. Des weiteren ist die Wasseraufnahme dieser Materialien ein exothermer Prozess, der zu einer starken Erhitzung und damit Zerstörung des Gewebes führen kann.

Zur Steigerung der Aufnahmekapazität für Körperflüssigkeiten, insbesondere von Blut, können Hämostatika mit pulverförmigen Superabsorbern versehen werden. Hierbei handelt es sich gewöhnlich um synthetische Polymere, beispielsweise um Methacrylcopolymerisate, welche eine beträchtliche Erhöhung der Absorption von Körperflüssigkeiten ermöglichen. Aus der DE 20 2005 020 566 U1 ist ein blutstillendes Mittel aus Polyurethanfasern bekannt, welches zusätzlich mit einem Methacrylcopolymerisatpulver versehen ist. Die aus der DE 20 2005 020 566 U1 bekannten Wundverbände bestehen im wesentlichen aus nicht resorbierbaren Komponenten, wodurch eine Applikation zur Blutstillung innerer Wunden ebenso wenig in Frage kommt.

Aus der US 2005/123588 A1 sind blutstillende Mittel mit Chitosan als Vliesmaterial und mikroporöse Polysaccharidmikrosphären bekannt.

In der WO 00/38752 A1 wird ein Wundvlies beschrieben, dass aus einem bioabbaubaren Trägermaterial besteht, welches mit einem rieselfähigen Fibrinklebergranulat beschichtet ist.

Gegenstand der GB 1 331 964 ist eine Absorptionslage ("absorbent sheet"), bei welchem die Fasern aus Cellulose und die Partikel unter anderem aus einem Cellulosederivat bestehen.

Die US 2005/095275 A1 betrifft ein Verbandmaterial, welches aus einem faserigen Vlies besteht. Das Vlies kann beispielsweise aus Kollagen bestehen. Außerdem kann das Verbandmaterial mikroporöse Polysaccharidmikrosphären aufweisen.

Die vorliegende Erfindung stellt sich somit die Aufgabe ein hämostatisches Mittel bereitzustellen, welches eine schnelle und wirksame Blutstillung ermöglicht und insbesondere auch zur Versorgung von inneren Blutungen, insbesondere parenchymatösen Organblutungen, eingesetzt werden kann.

Diese Aufgabe wird gelöst durch ein Vlies, insbesondere zur Blutstillung, mit einer feinen Faserstruktur aus Polymerfasern und gegenüber Körperflüssigkeiten saugfähigen Partikeln, wobei die Polymerfasern und/oder die Partikel aus einem resorbierbaren Material gebildet sind.

Unter einem resorbierbaren Material im Sinne der vorliegenden Erfindung sollen Materialien verstanden werden, welche entweder im Körper abgebaut oder in nicht abgebauter Form aus dem Körper insbesondere über die Niere ausgeschieden werden.

Unter Körperflüssigkeiten im Sinne der vorliegenden Erfindung sollen vor allem Blut und Wundwasser (Exsudat) verstanden werden.

Durch die Erfindung wird ein insbesondere blutstillendes Vlies bereitgestellt, welches sich aufgrund seiner Struktur in besonderem Maße zur Blutstillung eignet. Zum einen verfügt das erfindungsgemäße Vlies durch seine feine Faserstruktur über hohe Kapillarkräfte. Zum anderen zeichnet sich das Vlies gemäß der Erfindung durch ein hohes Oberflächen-Volumen-Verhältnis aus. Dadurch können Körperflüssigkeiten, insbesondere Blut, rasch und in ausreichendem Maße aufgesaugt werden. Das Aufnahme- bzw. Absorptionsvermögen von Körperflüssigkeiten wird im Falle des erfindungsgemäßen Vlieses zusätzlich dadurch erhöht, dass es saug- bzw. quellfähige Partikel aufweist. Durch das verbesserte Aufnahmevermögen für Körperflüssigkeiten kann eine besonders schnelle Blutstillung erzielt werden. Das erfindungsgemäße Vlies kann zudem ausschließlich aus resorbierbaren Materialien gebildet sein und eignet sich in dieser Beschaffenheit besonders zur Blutstillung von inneren Wunden.

In einer bevorzugten Ausführungsform liegt die Faserstruktur in unlyophilisierter Form vor. Im Vergleich zu den bei der Lyophilisation sich häufig ausbildenden grobblättrigen Vliesstrukturen weist die Faserstruktur des erfindungsgemäßen Vlieses eine verbesserte Resorbierbarkeit und insbesondere eine erhöhte Stabilität auf. Zudem bewirkt die faserförmige Struktur des Vlieses eine im Verhältnis zu den eher brüchigen Lyophilisaten höhere Flexibilität, wodurch ein verbessertes Handling des erfindungsgemäßen Vlieses ermöglicht wird.

Bei den Polymerfasern handelt es sich bevorzugt um gesponnene, insbesondere um elektrogesponnene, Polymerfasern. Besonderes bevorzugt handelt es sich bei den Polymerfasern um Mikrofasern. Dadurch werden einerseits besonders hohe Kapillarkräfte bei Kontakt mit Körperflüssigkeiten wirksam, wodurch die Blutstillung im zu versorgenden Wundareal beschleunigt werden kann. Andererseits bewirkt eine Mikrofaserstruktur eine Vergrößerung der Vliesoberfläche, wodurch die Aufnahme- bzw. Saugkapazität für Körperflüssigkeiten beschleunigt und insbesondere erhöht werden kann. Die Polymerfasern besitzen vorzugsweise einen Durchmesser zwischen 10 nm und 50 µm, insbesondere zwischen 100 nm und 25 µm. Besonders bevorzugt besitzen die Polymerfasern einen Durchmesser von 500 nm bis 1 µm.

Die saugfähigen Partikel können in gemahlener Form oder in Pulverform vorliegen. Bei den saugfähigen Partikeln kann es sich insbesondere um Granulatpartikel handeln. Die saugfähigen Partikel sind aus einem natürlichen Polymer hergestellt. Als natürliche Polymere kommen Proteine, vorzugsweise Kollagen und/oder Gelatine, in Betracht. Bevorzugt handelt es sich bei den saugfähigen Partikeln des erfindungsgemäßen Vlieses um Kollagen- und/oder Gelatine-partikel.

Besonders bevorzugt liegen die saugfähigen Partikel als Gelatinepartikel vor. Gelatine bildet in Gegenwart von Körperflüssigkeiten ein Hydrogel aus. Dies führt zu einer besonders effizienten Flüssigkeitsabsorption und im Falle von Blut zu einer schnellen Anreicherung von Blutplättchen, wodurch wiederum die Blutgerinnung beschleunigt wird.

In einer möglichen Ausführungsform sind die saugfähigen Partikel aus einer Mischung von Polymeren aufgebaut. Bezüglich der in Betracht kommenden Polymere wird auf die bisherige Beschreibung Bezug genommen.

Erfindungsgemäß kann es vorgesehen sein, dass die saugfähigen Partikel in lyophilisierter Form vorliegen. Die Anwesenheit von lyophilisierten Partikeln in der Vliesstruktur kann zu einer Vergrößerung der inneren Oberfläche des erfindungsgemäßen Vlieses führen, wodurch das Aufnahmevermögen für Körperflüssigkeiten zusätzlich verbessert werden kann.

In einer bevorzugten Ausführungsform weisen die saugfähigen Partikel ein Aufnahmevermögen für Körperflüssigkeiten auf, welches mindestens dem 10-fachen, insbesondere dem 20- bis 50-fachen, ihres jeweiligen Eigengewichtes entspricht. Besonders bevorzugt entspricht das Aufnahmevermögen für Körperflüssigkeiten mehr als dem 50-fachen des jeweiligen Partikeleigengewichtes.

Der Anteil der saugfähigen Partikel im erfindungsgemäßen Vlies beträgt vorzugsweise 0,1 bis 50 Gew.-%, insbesondere 1 bis 20 Gew.-% oder 10 bis 20 Gew.-%, bezogen auf das Gesamtgewicht des Vlieses. Erfindungsgemäß können die Partikel einen Durchmesser von 0,1 nm bis 1 µm, insbesondere 1 nm bis 500 nm, insbesondere 10 nm bis 500 nm, vorzugsweise 50 nm bis 250 nm, besitzen.

In einer weiteren vorteilhaften Ausführungsform weist das erfindungsgemäße Vlies chemisch unterschiedliche saugfähige Partikel auf. Beispielsweise kann das Vlies neben Gelatinepartikeln auch Kollagenpartikel aufweisen. Auf diese Weise können die hämostatischen Eigenschaften des erfindungsgemäßen Vlieses wegen der blutstillenden Eigenschaften von Kollagen zusätzlich verbessert werden.

Die Verteilung der saugfähigen Partikel in der Faserstruktur kann homogen oder inhomogen sein. Insbesondere können die Partikel entlang eines Gradienten in der Faserstruktur verteilt sein. Vorzugsweise sind die saugfähigen Partikel im wesentlichen homogen in der Faserstruktur verteilt. Die saugfähigen Partikel können mehr oder weniger lose in der Faserstruktur verteilt sein. Erfindungsgemäß können die Partikel insbesondere adhäsive Bindungen mit den Polymerfasern eingehen. Die adhäsiven Bindungen kommen vor allem auf der Basis von nicht kovalenten Bindungen, insbesondere Wasserstoffbrücken und/oder Van-der Waals-Kräften, zustande. Dadurch wird ein Wegspülen der Partikel bei Eintritt von Körperflüssigkeiten in das erfindungsgemäße Vlies verhindert.

In einer geeigneten Ausführungsform sind die saugfähigen Partikel zumindest teilweise, vorzugsweise vollständig, von den Polymerfasern ummantelt.

Bei dem resorbierbaren Material handelt es sich um ein Protein, vorzugsweise um Gelatine. Die Gelatine kann erfindungsgemäß bovinen, porcinen oder equinen Ursprungs sein. Gelatine equinen Ursprungs ist aufgrund der besonders geringen Infektionsrisiken bevorzugt. Weiterhin kann es sich bei dem resorbierbaren Material um rekombinant hergestellte Gelatine handeln. Beispielsweise kann die Gelatine aus Hefezellen gewonnen werden. Die Gelatine besitzt vorzugsweise ein Molekulargewicht (MG) von 100 kDa bis 500 kDa, insbesondere von 150 kDa bis 250 kDa. Die Gelatine kann insbesondere eine Bloomzahl von ca. 240 besitzen.

Die Polymerfasern und die saugfähigen Partikel sind aus dem gleichen resorbierbaren Material gebildet. Vorzugsweise bestehen die Polymerfasern und die saugfähigen Partikel aus Gelatine. Gelatine bildet bei Kontakt mit Körperflüssigkeiten eine hydrogelartige Beschaffenheit. Dadurch wandelt sich das erfindungsgemäße Vlies bei Kontakt mit Körperflüssigkeiten in eine gallertartige und flexible Masse um und lässt sich in diesem Zustand mit besonderem Vorteil auch an gekrümmte dreidimensionale Organoberflächen anmodellieren. Die gelartige flexible Masse haftet insbesondere gut auf organischen Geweben und versiegelt dadurch Wandoberflächen besonders gut und insbesondere flüssigkeitsdicht.

In einer vorteilhaften Ausführungsform weist das Vlies ein Aufnahmevermögen für Körperflüssigkeiten auf, welches dem 20- bis 100-fachen seines Eigengewichtes entspricht. Das erfindungsgemäße Vlies kann weiterhin eine maschenartige Struktur aufweisen. Bevorzugt weist das Vlies Maschen mit einem Abstand zwischen 10 µm und 150 µm, insbesondere zwischen 20 µm und 50 µm, auf.

Das Vlies liegt zweckmäßigerweise in flächiger Form, insbesondere als Wundverband, vor. Je nach Applikationsgebiet können unterschiedliche Vliesformen, insbesondere polygone Vliesformen, in Betracht kommen. Zum Beispiel können die Vliesformen viereckig oder sechseckig ausgebildet sein. Bevorzugt liegt das erfindungsgemäße Vlies in Quader-, Scheiben- oder Blütenformen vor.

In einer weiteren Ausführungsform ist das Vlies mit Additiven, insbesondere mit blutstillenden und/oder antimikrobiellen Substanzen, ausgerüstet. Bei den blutstillenden Substanzen kann es sich beispielsweise um Calciumsalze, insbesondere um Calciumchlorid, handeln. Calciumionen beschleunigen die Blutstillung, ohne die Saugfähigkeit des Vlieses zu beeinträchtigen. Als antimikrobielle Substanzen können Chitosan, Silbersalze und/oder elementares Silber verwendet werden. Im Fall von elementarem Silber kommt bevorzugt Nanosilber in Betracht.

Das erfindungsgemäße Vlies liegt zweckmäßigerweise in sterilisierter Form vor. Als Sterilisationsmethoden kommen alle fachmännischen Verfahren, beispielsweise Gamma-Sterilisierung, Ethylenoxidbegasung oder Plasmasterilisation in Betracht. Das erfindungsgemäße Vlies kann zudem in konfektionierter Form vorliegen.

Die Erfindung umfasst weiterhin ein Verfahren zur Herstellung eines Vlieses mit einer feinen Polymerfaserstruktur, insbesondere zur Blutstillung, wobei die Polymerfasern aus einer polymeren Fasermateriallösung hergestellt werden und während des Herstellens eine Dispersion, die gegenüber Körperflüssigkeiten saugfähige Partikel enthält, den sich bildenden Polymerfasern zugeführt wird. Das erfindungsgemäße Verfahren wird im Wege eines Einstufenprozesses durchgeführt, d.h. die Einlagerung der saugfähigen Partikel in die sich bildenden Polymerfasern erfolgt während der Faserherstellung.

In einer bevorzugten Ausführungsform werden die Polymerfasern durch Ausspinnen der Fasermateriallösung hergestellt. Als Spinntechniken kommen alle dem Fachmann geläufigen Techniken in Betracht. Bevorzugt werden die Polymerfasern durch Elektrospinnen hergestellt. Bei der Elektrospinntechnik wird eine hohe Spannung zwischen einer Spinndüse und einer Gegenelektrode angelegt. Die Lösung des zu verspinnenden Materials wird unter Druck durch die Spinndüse gepresst. Wenn das elektrische Feld die Oberflächenspannung der austretenden Tropfen überwindet, zieht es die tropfenförmigen Lösungen zu feinen Strahlen. Diese werden in Richtung Gegenkathode beschleunigt, durch die Luft gewirbelt und dabei kräftig gedehnt. Das Lösungsmittel verdunstet unter Ausbildung von sehr feinen Fasern, welche sich an der Gegenkathode abscheiden.

In einer möglichen Ausführungsform werden die Polymerfasern durch Versprühen, bevorzugt durch Electro-Spraying, der Fasermateriallösung hergestellt.

In einer bevorzugten Ausführungsform werden die Polymerfasern mit Hilfe zweier gleichzeitig arbeitender Düsen hergestellt. Eine Düse dient der Herstellung der reinen Polymerfasern. Die andere Düse dient der Zuführung der Suspension mit den saugfähigen Partikel an die sich während des Ausspinnprozesses bildenden Polymerfasern. Gegebenenfalls kann mit einer dritten Düse gearbeitet werden, mit welcher weitere Komponenten, insbesondere zur Compoundierung der Polymerfasern, zugeführt werden können. Als Komponenten können insbesondere weitere Polymere verwendet werden.

Zur Herstellung der Fasermateriallösung können alle dem Fachmann geläufigen Lösungsmittel verwendet werden. Bevorzugt werden protische Lösungsmittel, insbesondere Methan, Ethanol, Propanol und/oder iso-Propanol, verwendet. Als weitere Lösungsmittel kommen dipolar aprotische Lösungsmittel, beispielsweise DMSO, DMF, Acetonitril und/oder THF, in Frage. Ebenso können halogenierte, insbesondere chlorierte Kohlenwasserstoffe, beispielsweise Dichlormethan und/oder Chloroform, verwendet werden. Des weiteren sind fluorierte Lösungsmittel, insbesondere fluorierte Alkohole, denkbar. Bei den fluorierten Lösungsmitteln kann es sich beispielsweise um Pentafluorethanol und/oder Hexafluorpropanol handeln. Fluorierte Alkohole eignen sich insbesondere zum Versprühen von Gelatine.

Als mögliche Dispersionsmittel können grundsätzlich alle dem Fachmann bekannten organischen Lösungsmittel verwendet werden. Bevorzugt wird jedoch Wasser als Dispersionsmittel verwendet. Gegebenenfalls kann das Dispersionsmittel weitere Zusätze, insbesondere Salze, enthalten. Beispielsweise kann es sich bei einem entsprechend mit Zusätzen versehenen Dispersionsmittel um eine insbesondere physiologische Kochsalz-, Calciumchlorid- oder um eine Pufferlösung handeln. Besonders bevorzugt wird die Dispersion als Suspension, insbesondere als wässrige Suspension, hergestellt.

Weiterhin betrifft die Erfindung die Verwendung des erfindungsgemäßen Vlieses zur Herstellung eines medizintechnischen Gegenstandes, insbesondere zur Behandlung von Luft- und/oder Flüssigkeitsleckagen im menschlichen und/oder tierischen Körper. Das Vlies kann zur Herstellung eines hämostatischen Mittels, vorzugsweise eines Wundverbandes, verwendet werden. Das erfindungsgemäße Vlies kann zur topologischen und/oder zur internen Blutstillung verwendet werden. Bevorzugt dient das Vlies zur Behandlung von inneren Wunden, insbesondere von parenchymatösen Geweben. Weiterhin eignet sich das erfindungsgemäße Vlies zur Behandlung von Organblutungen, vorzugsweise von Organblutungen im Abdominalbereich. Des weiteren kann das Vlies zum Verschluss von Flüssigkeitsleckagen, insbesondere bei Blutgefäßen, vorzugsweise bei Herzgefäßen, und/oder zum Verschluss von Luftleckagen, insbesondere bei der Lunge, verwendet werden. Als weitere medizintechnische Gegenstände kommen Implantate, beispielsweise Inkontinenzprodukte und/oder Hernienersatzimplantate, in Frage. Bei den Hernienersatz-implantaten handelt es sich gewöhnlich um Herniennetze. Bevorzugt sind die Hernieneratzimplantate aus nicht resorbierbaren Materialien, insbesondere aus Polypropylen, Polyurethan und/oder hochmodularem und nicht resorbierbarem Polyvinylalkohol, gebildet. Die nicht resorbierbaren Materialien können gegebenenfalls vernetzt, vorzugsweise chemisch vernetzt, sein. Als chemische Vernetzungsreagenzien kommt zum Beispiel Glutaraldehyd in Betracht. Weiterhin eignet sich das erfindungsgemäße Vlies als Implantat zur Abdichtung von Anastomosen, insbesondere von Darmanstomosen.

Das erfindungsgemäße Vlies kann außerdem als Stützstruktur, vorzugsweise als Stütz- und Überbrückungstruktur, für Tissue Engineering verwendet werden. Tissue Engineering beschäftigt sich allgemein der künstlichen Herstellung und Konditionierung von Gewebestrukturen, vor allem zur Anwendung in der regenerativen Medizin. Hierbei kommt der Tissue-Engineering-Leitstruktur eine besondere Bedeutung zu, da Zellen außerhalb ihrer natürlichen Umgebung nur eingeschränkt fähig sind, dreidimensionale Strukturen auszubilden. Das erfindungsgemäße Vlies dient als Stütz- bzw. Leitstruktur und ermöglicht die Erzeugung dreidimensionaler Gewebekonstrukte. Die kleinen Faserdurchmesser des Vlieses entsprechen vorzugsweise den Fasern des natürlichen Kollagens in der extrazellulären Matrix des Gewebes. Besonders bevorzugt eignen sich derartige Gewebekonstrukte zur Züchtung von knorpelbildenden Zellen.

Weiterhin kann das erfindungsgemäße Vlies als Kleber für ein Implantat, insbesondere für ein Herniennetz, verwendet werden.

Die Erfindung betrifft auch ein hämostatisches Mittel, insbesondere ein Wundverband, in Form eines Vlieses. Bezüglich der Einzelheiten und Merkmale des Vlieses wird auf die bisherige Beschreibung verwiesen.

Die Erfindung betrifft weiterhin ein Hernienersatzimplantat, vorzugsweise Herniennetz, insbesondere zur Bauchwandverstärkung, in Form eines Vlieses, wobei bezüglich der Einzelheiten und Merkmale des Vlieses auf die bisherige Beschreibung Bezug genommen wird.

Weitere Merkmale und Einzelheiten der Erfindung ergeben sich aus dem nachfolgenden Beispiel sowie der nachfolgenden Figurenbeschreibung.

### Herstellung eines Gelatinevliese mit Gelatinepartikeln

Zur Herstellung des Vlieses wird eine Lösung von Gelatine mit einem Molekulargewicht (MG) von ca. 150 kDa in Pentafluorethanol sowie eine wässrige Suspension von Gelatinepartikeln (MG ca. 150 kDa) mit einem

Durchmesser von 50 nm bis 250 nm hergestellt. Zur Herstellung der wässrigen Suspension wird eine wässrige Calciumchloridlösung verwendet. Anschließend wird die Gelatinelösung zusammen mit der wässrigen Gelatinesuspension zu einem Vlies im Wege des Elektrospinnens ausgesponnen. Die Herstellung des Vlieses erfolgt vorzugsweise mit Hilfe zweier gleichzeitig arbeitender Düsen, wobei die eine Düse dem Ausspinnen der Gelatinelösung zu Gelatinefasern und die andere Düse der Zuführung der Gelatinesuspension an die sich bildenden Gelatinefasern dient.

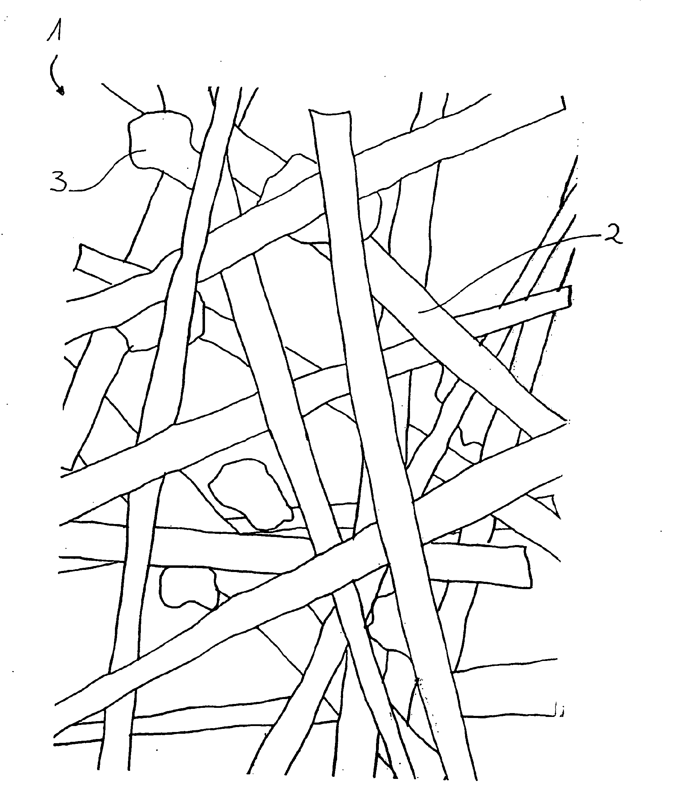

Die Figur zeigt schematisch die Struktur eines erfindungsgemäßen Vlieses **1.** Die Vliesstruktur **1** wird durch feine Polymerfasern **2** aus Gelatine gebildet. Die Gelatinefasern **2** besitzen bevorzugt einen Durchmesser zwischen 500 nm und **1** µm. Durch die feine Faserstruktur zeichnet sich das Vlies **1** insgesamt durch hohe Kapillarkräfte und insbesondere ein hohes Oberflächen-Volumen-Verhältnis aus. Auf diese Weise können Blutungen nach Auflage des Vlieses **1** auf ein Wundareal schnell und dauerhaft zum Stillstand gebracht werden. Zwischen den Gelatinefasern **2** befinden sich saug- bzw. quellfähige Gelatinepartikel **3,** welche das Aufnahmevermögen für Blutflüssigkeit zusätzlich erhöhen. Die Gelatinepartikel **3** (in der Figur vergrößert dargestellt) besitzen bevorzugt einen Durchmesser zwischen 50 nm und 250 nm. Die Gelatinepartikel **3** sind über adhäsive Bindungen an die Gelatinefasern **2** fixiert. Dadurch werden die Gelatinepartikel **3** bei Eintritt von Blutflüssigkeit nicht weggespült. Im Fall von Gelatine handelt es sich um ein körperverträgliches Material, welches bei Kontakt mit Körperflüssigkeiten in einen gallertartigen und flexiblen Zustand übergeht. In diesem Zustand lässt sich Gelatine mit besonderem Vorteil auch an nicht ebene Wundtopologien anmodellieren. Insgesamt sorgen daher die feine Gelatinefaserstruktur des Vlieses **1** sowie die quellfähigen Gelatinepartikel **3** für eine gute Aufnahmekapazität von Blutflüssigkeiten, so dass auch stark blutende oder exsudierende Wunden erfolgreich mit Hilfe des Vlieses **1** behandelt werden können.

## Patentansprüche

1. Vlies, insbesondere zur Blutstillung, mit einer feinen Faserstruktur aus Polymerfasern und gegenüber Körperflüssigkeiten saugfähigen Partikeln, **dadurch gekennzeichnet, dass** die Polymerfasern und die Partikel aus dem gleichen resorbierbaren Material gebildet sindund es sich bei dem resorbierbaren Material um ein Protein handelt.

2. Vlies nach Anspruch 1, **dadurch gekennzeichnet, dass** die Faserstruktur in unlyophilisierter Form vorliegt.

3. Vlies nach Anspruch 1 oder 2, **dadurch gekennzeichnet, dass** es sich bei den Polymerfasern um Mikrofasern handelt.

4. Vlies nach einem der vorhergehenden Ansprüche, **dadurch gekennzeichnet, dass** die Polymerfasern einen Durchmesser zwischen 10 nm und 50 µm, insbesondere zwischen 100 nm und 25 µm, vorzugsweise 500 nm und 1 µm, besitzen.

5. Vlies nach einem der vorhergehenden Ansprüche, **dadurch gekennzeichnet, dass** es sich bei den saugfähigen Partikeln um Polymerpartikel handelt.

6. Vlies nach einem der vorhergehenden Ansprüche, **dadurch gekennzeichnet, dass** die saugfähigen Partikel in lyophilisierter Form vorliegen.

7. Vlies nach einem der vorhergehenden Ansprüche, **dadurch gekennzeichnet, dass** die saugfähigen Partikel ein Aufnahmevermögen für Körperflüssigkeiten aufweisen, das mindestens dem 10- fachen, insbesondere dem 20- bis 50-fachen, ihres jeweiligen Eigengewichtes entspricht.

8. Vlies nach einem der vorhergehenden Ansprüche, **dadurch gekennzeichnet, dass** der Anteil der saugfähigen Partikel im Vlies 0,1 bis 50 Gew.-%, insbesondere 1 bis 20 Gew.-% oder 10 bis 20 Gew.-%, bezogen auf das Gesamtgewicht des Vlieses, beträgt.

9. Vlies nach einem der vorhergehenden Ansprüche, **dadurch gekennzeichnet, dass** die saugfähigen Partikel einen Durchmesser von 0,1 nm bis 1 µm, insbesondere 10 nm bis 500 nm, vorzugsweise 50 nm bis 250 nm besitzen.

10. Vlies nach einem der vorhergehenden Ansprüche, **dadurch gekennzeichnet, dass** die saugfähigen Partikel zumindest teilweise, vorzugsweise vollständig, von den Polymerfasern ummantelt sind.

11. Vlies nach einem der vorhergehenden Ansprüche, **dadurch gekennzeichnet, dass** es sich bei dem resorbierbaren Material um Gelatine handelt.

12. Vlies nach Anspruch 11, **dadurch gekennzeichnet, dass** die Gelatine ein Molekulargewicht von 100 kDa bis 500 kDa, insbesondere 150 kDa bis 250 kDa, besitzt.

13. Vlies nach einem der vorhergehenden Ansprüche, **dadurch gekennzeichnet, dass** das Vlies ein Aufnahmevermögen für Körperflüssigkeiten aufweist, das dem 20- bis 100-fachen seines Eigengewichtes entspricht.

14. Verfahren zur Herstellung eines Vlieses mit einer feinen Polymerfaserstruktur nach einem der vorhergehenden Ansprüche, wobei die Polymerfasern aus einer polymeren Fasermateriallösung hergestellt werden und während des Herstellens eine Dispersion, die die gegenüber Körperflüssigkeiten saugfähigen Partikel enthält, den sich bildenden Polymerfasern zugeführt wird.

15. Verwendung eines Vlieses nach einem der Ansprüche 1 bis 13 zur Herstellung eines medizintechnischen Gegenstandes, insbesondere eines hämostatischen Mittels, oder als Stütz- und Überbrückungsstruktur für Tissue Engineering.

16. Herniennetz, insbesondere zur Bauchwandverstärkung, umfassend ein Vlies nach einem der Ansprüche 1 bis 13.

## Claims

1. Non-woven fabric, in particular for haemostasis, comprising a fine fibrous structure composed of polymer fibres and particles capable of absorbing bodily fluids, **characterized in that** the polymer fibres and the particles are formed of the same resorbable material and the resorbable material is a protein.

2. Non-woven fabric according to Claim 1, **characterized in that** the fibrous structure is in unlyophilized form.

3. Non-woven fabric according to Claim 1 or 2, **characterized in that** the polymer fibres are microfibres.

4. Non-woven fabric according to any preceding claim, **characterized in that** the polymer fibres have a diameter between 10 nm and 50 µm, in particular between 100 nm and 25 µm, preferably 500 nm and 1 µm.

5. Non-woven fabric according to any preceding claim, **characterized in that** the particles capable of absorbing bodily fluids are polymer particles.

6. Non-woven fabric according to any preceding claim, **characterized in that** the particles capable of absorbing bodily fluids are in lyophilized form.

7. Non-woven fabric according to any preceding claim, **characterized in that** the particles capable of absorbing bodily fluids have an absorbance for bodily fluids which corresponds to at least 10 times, in particular 20 to 50 times, their respective own weight.

8. Non-woven fabric according to any preceding claim, **characterized in that** the proportion in the non-woven fabric of particles capable of absorbing bodily fluids is 0.1 to 50 wt%, in particular 1 to 20 wt% or 10 to 20 wt%, based on the overall weight of the non-woven fabric.

9. Non-woven fabric according to any preceding claim, **characterized in that** the particles capable of absorbing bodily fluids have a diameter of 0.1 nm to 1 µm, in particular 10 nm to 500 nm, preferably 50 nm to 250 nm.

10. Non-woven fabric according to any preceding claim, **characterized in that** the particles capable of absorbing bodily fluids are at least partly, preferably wholly, ensheathed by the polymer fibres.

11. Non-woven fabric according to any preceding claim, **characterized in that** the resorbable material is gelatin.

12. Non-woven fabric according to Claim 11, **characterized in that** the gelatin has a molecular weight of 100 kDa to 500 kDa, in particular 150 kDa to 250 kDa.

13. Non-woven fabric according to any preceding claim, **characterized in that** the non-woven fabric has an absorbance for bodily fluids which corresponds to 20 to 100 times its own weight.

14. Process for producing a non-woven fabric having a fine polymeric fibrous structure according to any preceding claim, wherein the polymer fibres are produced from a polymeric solution of fibre material and a dispersion which contains particles capable of absorbing bodily fluids is added to the nascent polymer fibres as they are being produced.

15. Use of a non-woven fabric according to any of Claims 1 to 13 in the manufacture of a medical device, in particular a haemostatic means, or as a supporting and bridging structure for tissue engineering.

16. Hernia mesh, in particular for abdominal wall reinforcement, comprising a non-woven fabric according to any of Claims 1 to 13.

## Revendications

1. Etoffe non-tissée, en particulier pour l'hémostase, avec une fine structure fibreuse en fibres de polymère et des particules absorbantes à l'égard des liquides corporels, **caractérisée en ce que** les fibres de polymère et les particules sont formées de la même matière résorbable, et la matière résorbable est une protéine.

2. Etoffe non-tissée selon la revendication 1, **caractérisée en ce que** la structure fibreuse se présente sous forme non lyophilisée.

3. Etoffe non-tissée selon la revendication 1 ou 2, **caractérisée en ce que** les fibres de polymère sont des microfibres.

4. Etoffe non-tissée selon l'une quelconque des revendications précédentes, **caractérisée en ce que** les fibres de polymère présentent un diamètre compris entre 10 nm et 50 µm, en particulier entre 100 nm et 25 µm, de préférence entre 500 nm et 1 µm.

5. Etoffe non-tissée selon l'une quelconque des revendications précédentes, **caractérisée en ce que** les particules absorbantes sont des particules de polymère.

6. Etoffe non-tissée selon l'une quelconque des revendications précédentes, **caractérisée en ce que** les particules absorbantes se présentent sous forme lyophilisée.

7. Etoffe non-tissée selon l'une quelconque des revendications précédentes, **caractérisée en ce que** les particules absorbantes présentent une capacité d'absorption pour des liquides corporels, qui correspond à au moins 10 fois, en particulier au moins 20 à 50 fois, leur poids propre respectif.

8. Etoffe non-tissée selon l'une quelconque des revendications précédentes, **caractérisée en ce que** la proportion des particules absorbantes dans l'étoffe non-tissée vaut 0,1 à 50 % en poids, en particulier 1 à 20 % en poids ou 10 à 20 % en poids, rapportée au poids total de l'étoffe.

9. Etoffe non-tissée selon l'une quelconque des revendications précédentes, **caractérisée en ce que** les particules absorbantes présentent un diamètre de 0,1 nm à 1 µm, en particulier de 10 nm à 500 nm, de préférence de 50 nm à 250 nm.

10. Etoffe non-tissée selon l'une quelconque des revendications précédentes, **caractérisée en ce que** les particules absorbantes sont au moins partiellement, de préférence entièrement, enveloppées par les fibres de polymère.

11. Etoffe non-tissée selon l'une quelconque des revendications précédentes, **caractérisée en ce que** la matière résorbable est la gélatine.

12. Etoffe non-tissée selon la revendication 11, **caractérisée en ce que** la gélatine présente un poids moléculaire de 100 kDa à 500 kDa, en particulier de 150 kDa à 250 kDa.

13. Etoffe non-tissée selon l'une quelconque des revendications précédentes, **caractérisée en ce que** l'étoffe présente une capacité d'absorption pour des liquides corporels, qui correspond à 20 à 100 fois son poids propre.

14. Procédé de fabrication d'une étoffe non-tissée avec une fine structure de fibres de polymère selon l'une quelconque des revendications précédentes, dans lequel on fabrique les fibres de polymère à partir d'une solution de matière de fibres polymères et, pendant la fabrication, on ajoute aux fibres de polymère qui se forment, une dispersion qui contient des particules absorbantes à l'égard des liquides corporels.

15. Utilisation d'une étoffe non-tissée selon l'une quelconque des revendications 1 à 13 pour la fabrication d'un objet médical, en particulier d'un moyen hémostatique, ou comme structure de soutien ou de pontage pour l'ingénierie tissulaire.

16. Filet pour hernie, en particulier pour le renforcement de la paroi abdominale, comprenant une étoffe non-tissée selon l'une quelconque des revendications 1 à 13.
